# EUROPEAN PATENT SPECIFICATION

(11) **EP 2 874 663 B1**
(45) Date of publication and mention of the grant of the patent: **10.01.2018**
(21) Application number: 13773359.8
(22) Date of filing: 19.07.2013
(51) Int. Cl.: A61K 47/50, A61K 49/18, A61P 29/00

(54) **NANOCONSTRUCTS WITH PHARMACOLOGICAL ACTIVITY**
NANOKONSTRUKTE MIT PHARMAKOLOGISCHER WIRKUNG
NANOCONSTRUCTIONS POSSÉDANT UNE ACTIVITÉ PHARMACOLOGIQUE

(30) Priority: 19.07.2012 IT RM20120350
(43) Date of publication of application: 27.05.2015
(73) Proprietor: Università degli Studi di Milano - Bicocca, 20126 Milano (IT); Universita' degli Studi di Milano, 20122 Milano (IT)
(72) Inventor: GRANUCCI, Francesca, 20090 Trezzano sul Naviglio MI (IT); PROSPERI, Davide, 20158 Milano MI (IT); ZANONI, Ivan, 20134 Milano MI (IT); CORSI, Fabio, Ruggero, Maria, 20090 Buccinasco MI (IT); COLOMBO, Miriam, 20142 Milano MI (IT)
(74) Representative: Di Giovine, Paolo
(86) International application number: PCT/IB2013/055943
(87) International publication number: WO 2014/013473

(56) References cited:
- WO-A1-2007/097593
- US-A1- 2005 136 258
- CHOO E. S. G. ET AL.: "Controlled loading of superparamagnetic nanoparticles in fluorescent nanogels as effective T2-weighted MRI contrast agents", JOURNAL OF MATERIAL CHEMISTRY, vol. 21, 15 December 2010 (2010-12-15), pages 2310-2319, XP002692455,
- CHENG-AN J. L. ET AL.: "Design of an Amphiphilic Polymer for Nanoparticles Coating and Functionalization", SMALL, vol. 4, no. 3, 13 February 2008 (2008-02-13), pages 334-341, XP002692454, cited in the application

## Description

The present invention relates to methods and means of transport of active substances in the cell cytosol. In particular, the invention relates to means that make it possible to transport directly in the cytosol substances capable of interacting with protein systems, such as the family of nuclear factors of activated T-cells (NFATs) or nucleic acids localized in the cytosol. In particular, the invention relates to nanoconstructs useful in the treatment of inflammatory diseases, methods for their preparation, and compositions containing them.

### PRIOR ART

### Cytosol

Cytosol is the soluble fraction of the cytoplasm of cell organelles, formed by a solution of mineral salts, organic molecules, macromolecules and proteins. Biochemical reactions of fundamental importance for the physiology of the cell and of living organisms in general take place in this cell compartment. The reactions that take place in the cytosol here include the reaction of dephosphorylation of the NFAT protein from the Ca²⁺/calmodulin-dependent phosphatase calcineurin (Cn) protein.

### NFAT

The NFAT protein means a member of a family of transcription factors constituted by the members NFAT1 (NFATc2), NFAT2 (NFATc1), NFAT3 (NFATc4) and NFAT4 (NFATc3), and the various isoforms thereof. The proteins of the NFAT family are evolutionarily linked to the REL-NF-kB family of transcription factors. The NFAT proteins were originally identified as transcription factors able to regulate the production of IL-2 in the T-cells, although various functions of NFAT with regard to adaptive immunity have since been identified, including the differentiation and activation of B- and T-cells.

NFAT is activated in neutrophils, macrophages and dendritic cells (DCs) in response to Curdian, but is also activated in DCs in response to LPS and is regulated by TNFα in the macrophages.

The activity of isoforms 1-4 is controlled by calcineurin (Cn), a Ca²⁺/calmodulin-dependent phosphatase which, once activated, increases the intracellular Ca²⁺ concentration and dephosphorylates the phosphorylation motifs present at the N-terminal of NFAT, expressing a nuclear sequence that determines the nuclear import of NFAT. The NFAT proteins interact with partner proteins referred to generically as NFATn to produce active NFAT transcription factor complexes and to control, in subsequent analyses, the production of cytokines.

Chronic and inflammatory autoimmune diseases as well as problems linked to transplant rejection are commonly treated using immunosuppressive drugs, including cyclosporin A (CsA) and FK506, which are powerful inhibitors of the gene transcription of cytokines in activated immune cells. CsA and FK506 inhibit the activity of Cn. However, they have very pronounced side effects. CsA is responsible for vascular lesions of small vessels, in particular inside the kidneys, and of large vessels due to hypertension and hyperlipidemia, whereas FK506 can lead to nephrotoxicity, neurotoxicity, diabetes mellitus and hypertension. The high level of toxicity of these drugs is caused by the fact that Cn is expressed ubiquitously and controls not only the activation of NFAT, but also other transcription factors.

There is thus a need for new, less toxic immunosuppressive drugs that, whilst inhibiting specifically the NFAT precursor, inhibit the expression of key inflammatory molecules required for the activation of the immune response.

### VIVIT

Peptides with the sequence Pro-X-Ile-X-Ile-Thr (PxlxlT), derived from the hidden Cn-bonding motif found in NFAT proteins, compete with NFAT for the bond to the Cn, thus compromising the bond and dephosphorylation of NFAT as demonstrated in enzyme assays. In particular, it has been demonstrated that the liogopeptide Val-Ile-Val-Ile-Thr (VIVIT) in the 16mer form has an increased affinity for Cn and effectively inhibits NFAT and its activity, but not the activity of the NF-kB transcription factor, as indicated by the activation of the respective gene reporters (Aramburu et al. Science. 1999). The VIVIT peptide is therefore more selective than CsA and FK506, which inhibit the activation of both transcription factors. In fact, patent application WO2008156363 describes various constructs comprising a VIVIT sequence for use in the generic treatment of inflammation.

It is, however, well known that the peptide material is unable to pass independently through the cell membrane in order to transfer directly into the cytosol, and in any case there are well-known problems regarding *in vivo* stability, which limit the use of such material. Results reported in the experimental part of the present application have confirmed that peptides comprising the VIVIT sequence are not able to reach the cell cytosol, where the target Cn protein is localized, in an amount sufficient to be effective as inhibitors of inflammatory processes (Sieber et al. Cell communication and signaling: CCS. 2009).

**Nanoparticles.** It is known that solid particles having dimensions in the nanoscale range constitute a valid vehicular system for the transport of active molecules. Once bonded to the nanoparticles, such molecules are protected from *in vivo* degradation and can be transported and released inside the cell.

The majority of the nanoparticles used in the therapeutic/diagnostic sector can be classified into two main classes: particles that contain organic molecules as the main component, and those that use inorganic elements, usually metals, as the nucleus. The particles belonging to the last group have numerous advantages, including: 1) the possibility of utilizing the crystalline nucleus in order to obtain a relevant physical property, 2) the inorganic nucleus enables effective control of the dimensional and morphological characteristics of the final nanoconstruct.

The majority of inorganic commercial nanoparticles share the same basic structure: a central nucleus of spherical, cubical or anisotropic morphology, which defines the physical properties (including fluorescence, optical, magnetic and electronic properties of the particle), with a protective organic coating on their surface. This coating protects the nucleus from *in vivo* physiological degradation and allows the formation of electrostatic or covalent bonds to organic (bio)molecules having functional groups that can be used for the bond.

The problem posed by nanoparticles that can be used as carriers of biologically active substances lies in the fact that such nanoconstructs are internalized in the cell by means of the endosomal pathway and are directed to the process of liposomal degradation. In this sense, the biologically active substance is degraded and therefore does not have the option to reach the cytosol in a pharmacologically active amount.

International patent application WO2005065081 describes the preparation of magnetic nanoparticles comprising an amphiphilic coating that can also be used as carriers of active molecules. The document describes the use of such nanoparticles in diagnostic methods or therapeutic treatments for tumors. However, no evaluation of the target cells of such particles has been carried out by the authors of the document.

International patent application WO2007097593 discloses water soluble magnetic nanocomposite using an amphiphilic compound. Specifically discloses water soluble magnetic nanocomposite which may be used as a contrast agent for magnetic resonance imaging (MRI) and a drug delivery system for simultaneous diagnosis and treatment.

The patent application US2005136258 discloses nanostructures, methods of preparing nanostructures, methods of detecting targets in subjects, and methods of treating diseases in subjects, are disclosed. The nanostructure includes a quantum dot and a hydrophobic protection structure. The hydrophobic protection structure includes a capping ligand and an amphiphilic copolymer, where the hydrophobic protection structure encapsulates the quantum dot.

The object of the present invention is that of avoiding the above-mentioned disadvantages in the prior art systems.

### SUMMARY OF THE INVENTION

The present invention is based on the unexpected discovery that nanoparticles comprising an amphiphilic polymer-based coating are able to transport and release an active molecule directly in the cell cytosol. From the cytosol, the molecule can then also diffuse into the nucleus. Without wanting to limit the scope of the invention to specific scientific theories, this result is likely achieved at least in part thanks to the ability of the nanoconstruct, referred to hereinafter as MYTS, to pass directly through the cell membrane and in part thanks to the ability to escape the traditional endosomal system and therefore the lyosomal degradation pathway.

An object of the present invention is a nanoconstruct according to claim 1.

A further object of the present invention is a pharmaceutical composition comprising the nanoconstructs according to claim 1 and/or one or more pharmaceutically acceptable excipients for use in the treatment of a disease or of an inflammatory state.

A fourth object of the present invention is a method for the preparation of a nanoconstruct according to claim 1, comprising the following steps:
a) prearranging a solution/suspension of inorganic nanoparticles with an external coating of an amphiphilic polymer activated by a linker;
b) adding to said solution/suspension one or more active molecules, letting it react with said linker.

### BRIEF DESCRIPTION OF THE FIGURES

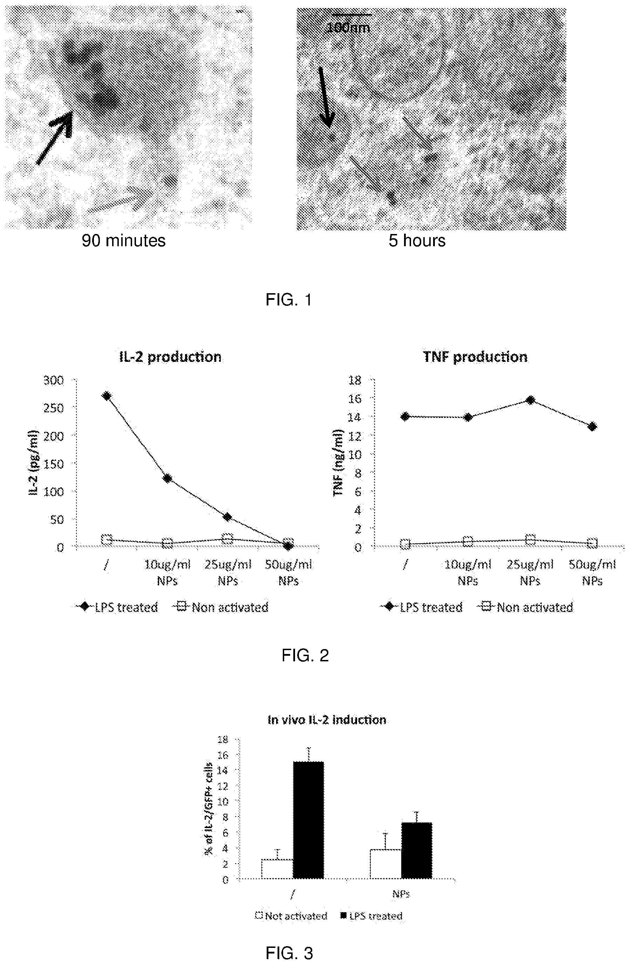

Figure 1. TEM analyses of DCs after exposure with the MTYS nanoconstructs. DCs derived from mouse bone marrow were co-cultivated with the nanoconstructs for five hours and were then analyzed by means of TEM. The black arrow indicates MYTS within the endosomes, the grey arrow indicates MYTS that escape the endosomes and enter the cytosol.
Figure 2. Inhibition of the transduction of the NFAT signals by means of the MYTS-VIVIT nanoconstructs in DCs derived from bone marrow. The DCs were pretreated, or not, with the indicated quantities of MYTS-VIVIT for two hours and then activated with LPS for 18 hours. The quantities of IL-2 and TNFα in the supernatant were measured using the ELISA method.
Figure 3. The NFAT pathway is active in the neutrophils. Mice genetically modified by IL-2/GFP were treated, or not, with MYTS-VIVIT and were then injected intravenously two hours after with LPS. The bars represent the percentage of GFP⁺ Ly6G⁺ spleen neutrophils three hours after the treatment with LPS. In this mouse model, GFP was inserted in the second exon of IL-2 and therefore has the same regulation of IL-2.

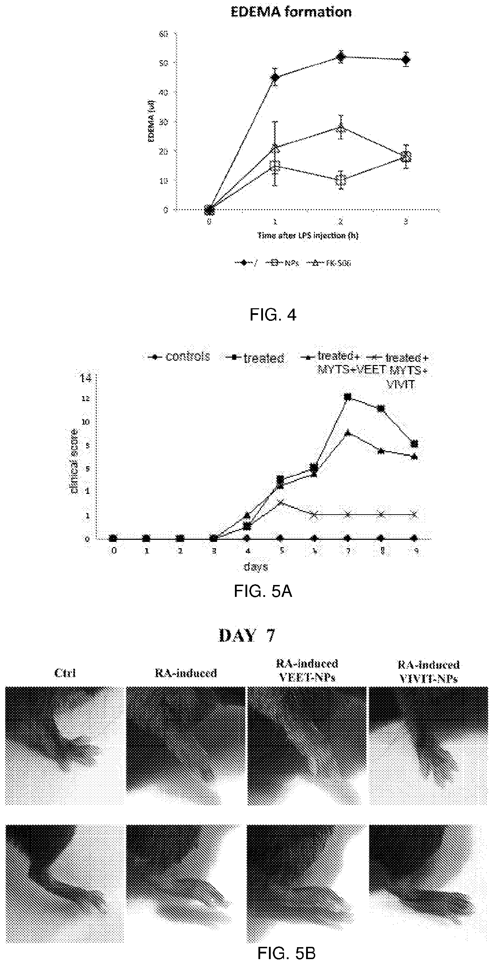

Figure 4. Inhibition of the NFAT pathway in the formation of edema of the paw in response to LPS. The animals were pretreated, or not, with the nanoconstructs (MYTS-VIVIT) or FK-506 (approximately 2 h) and were then treated locally with LPS. The edema was measured at the points in time indicated in the figure.
Figure 5. Inhibition of the development of arthritis. Figure 5A: the mice were treated endovenously, or were not treated, at -1, 0, +1, +3, +6, +9, and + 12 days with the nanoconstructs (MYTS-VIVIT) or with nanoconjugates MYTS-VEET (a peptide similar to VIVIT in which the sequence has been modified, thus losing specificity for Cn). Day 0 represents the start of the procedure of inducing arthritis. The clinical points indicative of the severity of the arthritis were assessed on the days indicated. In figure 5B, photographs showing arthritis on the seventh day in a treated mouse and in an untreated mouse are shown.

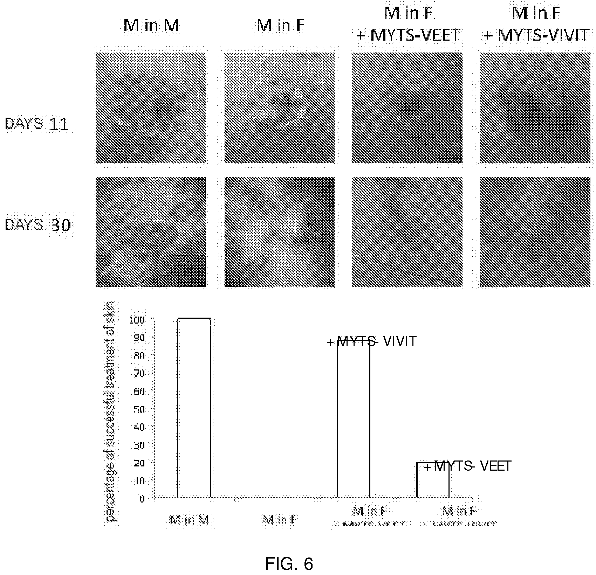

Figure 6. Inhibition of skin transplant rejection. Male skin was grafted in female mice. The mice were then injected endovenously, or not, with the nanoconstructs (MYTS-VIVIT or MYTS-VEET) on days -1, 0, +1, +3, +6, +9, +12, and +15. In addition, the mice were then left without treatment for a further 20 days. The boxes M in M represent the untreated controls and show the skin transplant from male mouse to male mouse. As can be seen, the graft is clearly accepted. The boxes M in F represent the skin transplant from male mouse to female mouse without treatment with the nanoconstructs. The scar of the rejection is clearly visible on day 30. The rejection can also be seen in boxes M in F (male into female) treated with the control MYTS-VEET nanoconstructs. Vice versa, in the boxes representing the transplant from male to female with MYTS-VIVIT treatment (M in F + MYTS-VIVIT), the acceptance of the graft is clearly visible. The bar chart beneath represents a quantification of the results of the experiment. To summarize, the transplant was accepted in 80% of cases treated with the MYTS-VIVIT nanoconstructs, whereas it was rejected in 100% of untreated cases and in 80% of cases treated with the control MYTS-VEET nanoconstruct.

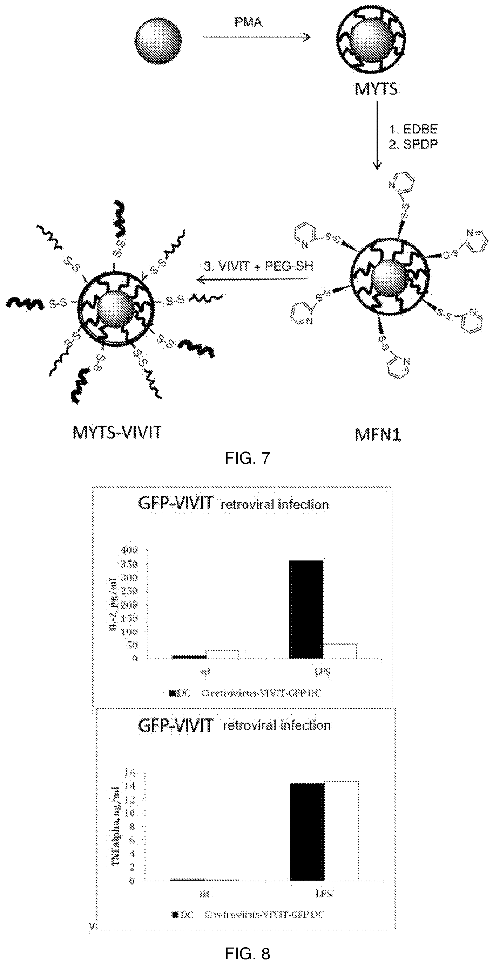

Figure 7. Schematic representation of the method for preparing the MYTS-VIVIT nanoconstructs in accordance with a specific embodiment described in detail in the examples.
Figure 8. Production of cytokines from dendritic cells transduced, or not, with the peptide of sequence SEQ ID NO:1 in response to LPS. DCs transduced with the peptide with sequence SEQ ID NO:1 fused to the GFP protein and non-transduced DCs were stimulated with LPS (100 ng/ml) for 18 hours. The production of IL-2 (NFAT-dependent, top box) and of TNF-α (NFAT-independent, bottom box) was then assessed by means of ELISA assay. It is noted that the expressions of the peptide with sequence SEQ ID NO:1 reduces the production of IL-2.

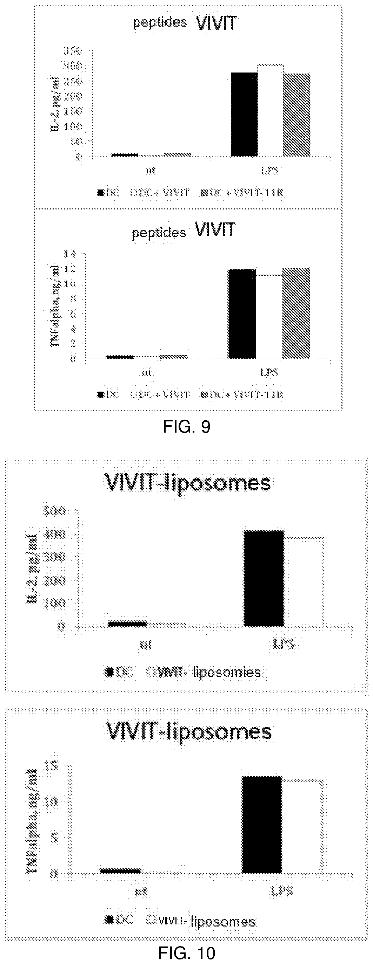

Figure 9. The unmodified peptide with sequence SEQ ID NO:1 or the same peptide with an 11-arginine tail is unable to penetrate into the cytosol of the DCs and to block the activity of NFATs. DCs derived from bone marrow were pretreated, or not, with the peptide with sequence SEQ ID NO:1 (25 uM) or with the peptide conjugated with an 11-arginine tail (25 uM) before being stimulated for 18 hours with 100 ng/ml of LPS. The release of IL-2 (top box) and of TNF-α (bottom box) in the supernatant was then assessed by ELISA assay. It is noted that neither the unmodified peptide nor the peptide modified with the 11-arginine tail was able to inhibit the activity of NFATs.
Figure 10. The peptide with sequence SEQ ID NO:1 comprised in the liposomes is unable to penetrate into the cytosol of DCs and block the activity of NFATs. DCs derived from bone marrow were pretreated, or not, with the phospholipid-based peptide included in liposomes before being stimulated for 18 hours with 100 ng/ml of LPS. The release of IL-2 (top box) and of TNF-α (bottom box) in the supernatant was then assessed by means of ELISA assay. It is noted that the peptide comprised in the liposomes was unable to inhibit the activity of NFATs.

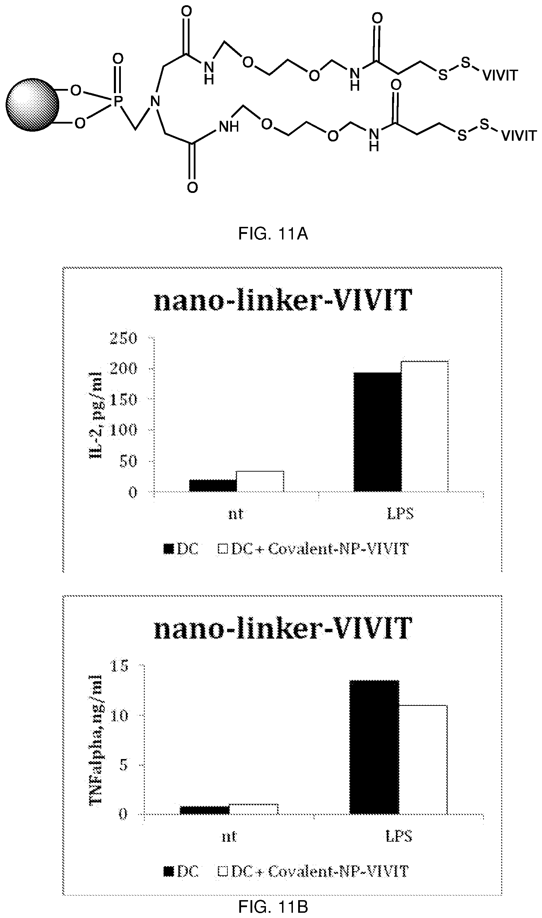

Figure 11. (A) The nanoparticle is formed of a magnetic crystalline magnetite core measuring approximately 8 nm in diameter. The N-phosphonomethylimminodiacetic acid (PMIDA) is used to introduce a site of immobilization for the organic compound on the iron oxide utilizing the increased infinity of the phosphonate group for the surface Fe-OH on the nanoparticle. The two carboxylic groups are converted into amino groups by means of condensation with the bifunctional diamino linker 2,2-(ethylenedioxy)bis(ethylamine) (EDBE) by activation with N-hydroxysuccinimidil esters (NHS). Then, the two amino groups are converted into pyridylthio propionate (PDP) groups reactive to thiols by means of addition of N-succinimidil-3[2-pyridylthio]-propionate (reagent SPDP). Lastly, the final nanoconstruct is obtained by reacting the nanoconjugate with the same peptide used in the MYTS-VIVIT nanoconstruct (sequence SEQ ID NO:1). (B) DCs derived from bone marrow were pretreated, or not, with the peptide directly bound to the magnetic nanoparticles (a concentration that corresponds to having the peptide 25 µM was used) before being stimulated for 18 hours with 100 ng/ml of LPS. The release of IL-2 (top box) and of TNF-α (lower box) in the supernatant was then assessed by means of ELISA assay. It is noted that the peptide directly bound to the nanoparticles was unable to inhibit the activity of NFATs.

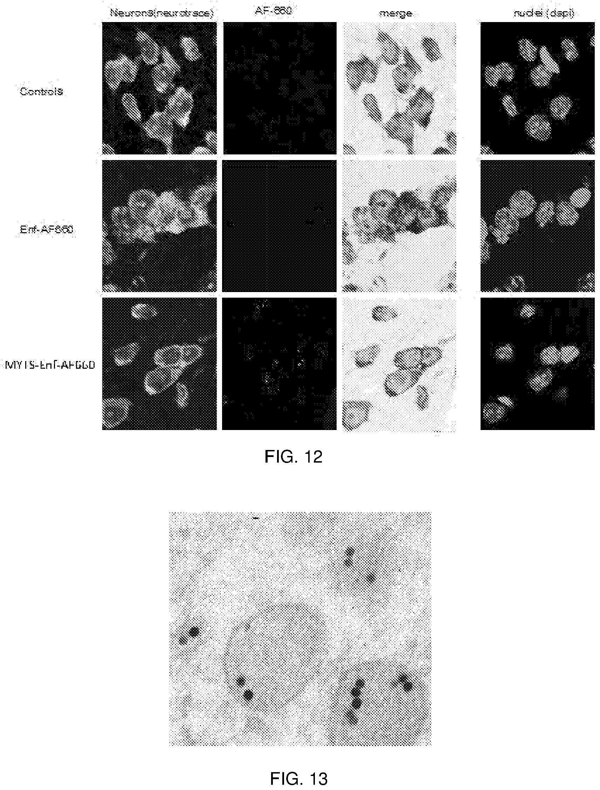

Figure 12. Cytosolic internalization and localization of the MYTS-enfuvirtide nanoconstruct. In the images shown here, the localization of the antiretroviral drug labeled with fluorescent probe within the cytosols of neurons obtained from cryosections of mouse brain extracts treated with MYTS-enfuvirtide can be clearly seen. The spots of fluorescence corresponding to neurons can be clearly seen only in the case of enfuvirtide conjugated to MYTS (row 3 at the bottom, fluorescence channel), whereas traces of fluorescence cannot be seen in the control and in the samples extracted from mice treated with the labeled drug not conjugated to the nanoparticles (rows 1 and 2 respectively)
Figure 13. TEM analyses of DCs after exposure with MYTS-polyethylene glycol nanoconstructs. The experiment demonstrates the localization in cytosol of MYTS polyethylene glycols (PM_{PEG} = 2000 Da). In the image, nanoconstructs conjugated with PEG₂₀₀₀ leaked from the endosomes can be seen, whilst the endosomes themselves are intact.

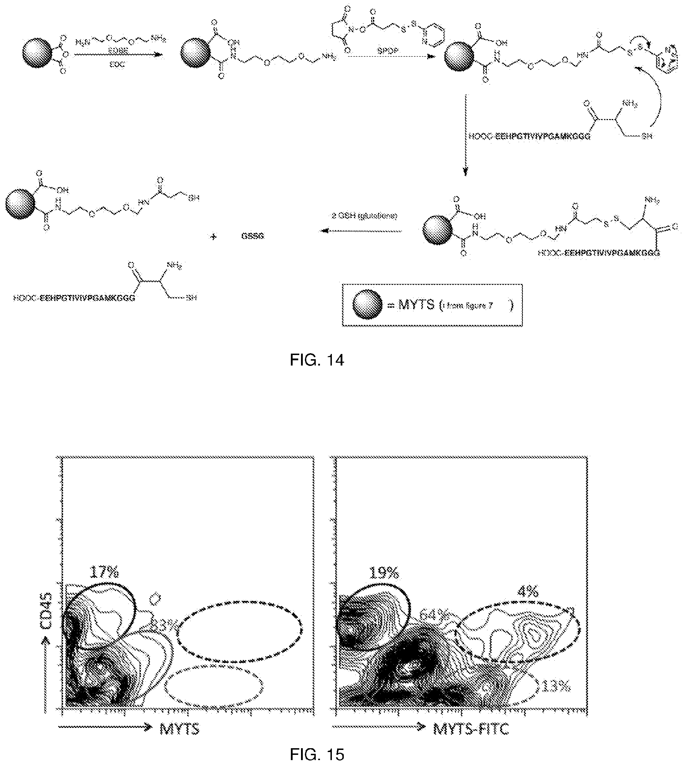

Figure 14. Schematic representation of the process of conjugation of the peptide with sequence SEQ ID NO:1 with the nanoconstruct by means of the EDBE-PDP linker in accordance with a preferred embodiment of the invention.
Figure 15. Dispersions at high concentration of nanoparticles labeled with fluorophore and unlabeled nanoparticles (MYTS-FITC and MYTS respectively) were incorporated in an amount approximately double that of the base cream and were then added to the rest of the base cream in successive dilutions, mixing until fully homogenized. The aforementioned preparations were then applied for three consecutive days to the skin of C57BL/6 mice shaved beforehand (area measuring 1 cm²). The mice were killed and a unicellular suspension was produced from the area of treated skin. The cells were then analyzed by means of FACS to determine the uptake in cells of hematopoietic origin (CD45+) and not (CD45-). As can be seen from the figure, the nanoparticles are absorbed by both groups of cells.

### DETAILED DESCRIPTION OF THE INVENTION

The present invention is based on the following aspects, described in detail below.

### Inorganic nanoparticle

The nanoparticles formed on an inorganic basis share the same basic structure: a central nucleus that defines the physical properties (including fluorescence and optical, magnetic and electronic properties of the particle) and optionally a protective organic coating on the surface. The organic coating, for example a hydrophobic surfactant that increases the solubility of the nanoparticles in suspension, is preferably of the long-chain type selected for example from oleic acid, oleylamine and trioctylphosphine.

The inorganic nanoparticles of the present invention generally have a metal or metal oxide nucleus, of crystalline or non-crystalline type. Suitable metals include, for example, gold, silver, iron, manganese, copper, nickel, platinum and palladium. By contrast, examples of metal oxides include γ-Fe₂O₃, Fe₃O₄, MnFe₂O₄, CoFe₂O₄, MnO, quantum dots (for example CdSe, CdS, CdSe/ZnS, etc.), TiO₂, SiO₂, and CeO₂.

Nanoparticles measuring between 5 and 50 nanometers are generally used.

Examples of nanoparticles that can be used in the present invention are prepared as described, for example, in in J. Park et al. (Nat. Mater. 2004, 3, 891-895), L. Polito et al. (J. Am. Chem. Soc. 2008, 130, 12712-12724), M. Brust et al. (J. Chem. Soc. Chem. Commun. 1994, 801-802), C. B. Murray et al. (J. Am. Chem. Soc. 1993, 115, 8706-8715), or are commercially available, for example, from: AC Diagnostics, Inc. (Fayettiville, AR; USA); SkySpring Nanomaterials, Inc. (Houston, TX; USA); Sigma-Aldrich (St. Louis, MO; USA); Life Technologies Ltd (Paisley, UK); and mkNANO (Missisauga, ON; Canada).

### Nanoparticle coating with an amphiphilic polymer

The method according to the invention envisages the use of a nanoconstruct comprising an inorganic nanoparticle or a nanoparticles formed as described above, having an outer coating formed by an amphiphilic polymer.

Examples of procedures for preparing inorganic nanoparticles with an outer coating of an amphiphilic polymer are described in detail in patent application WO2005065081, incorporated herein fully by way of reference. Such procedures envisage the formation of the amphiphilic coating by means of the use of a capping ligand and of an amphiphilic block copolymer. The amphiphilic coating is normally formed by block copolymers.

An exemplary, but non-limiting, list of capping ligands is provided on page 10, lines 21-30 and page 11, lines 1-3 of patent application WO2005065081.

An exemplary, but non-limiting, list of amphiphilic block copolymers is provided on pages 11 to 23 of patent application WO2005065081.

In one embodiment, the amphiphilic coating is obtained by condensation of poly(isobutylene-maleic anhydride) and dodecylamine (PMA), as described for example in C.-A. J. Lin et al. Small 2008, 3, 334-341.

### Molecule bound to the nanoparticle through said amphiphilic coating

The nanoconstruct of the present invention also comprises an active molecule bound to the nanoparticle through the amphiphilic coating.

Said molecule can be any molecule having an affinity for one or more target molecules localized in the cytosol. The expression "having an affinity for one or more target molecules localized in the cytosol" means that the molecule is able to interact with the target molecule in the cytoplasmic environment.

The molecule can be selected from, but is not limited to: peptides, proteins (for example monoclonal or polyclonal antibodies), sugars, synthetic polymers, polynucleotides, fatty acids, small organic molecules, or combinations thereof.

The word "polynucleotides" comprises DNA, RNA, siRNA and miRNA, whether of viral, bacterial, plant or animal (for example mammal) origin, and whether synthetic, single-strand or double-strand, comprising natural or non-natural or chemically modified nucleotides. In addition, the molecule may also include labeled molecules, for example molecules labeled radioactively or with fluorophores.

The active molecule can be a peptide selected from, but not limited to, Ac-Tyr-Thr-Ser-Leu-Ile-His-Ser-Leu-Ile-Glu-Glu-Ser-Gln-Asn-Gln-Gln-Glu-Lys-Asn-Glu-Gln-Glu-Leu-Leu-Glu-Leu-Asp-Lys-Trp-Ala-Ser-Leu-Trp-Asn-Trp-Phe-NH2 (enfuvirtide of SEQ ID NO:6), peptides that promote the penetration of substances in the nucleus (nuclear localization sequence), or a peptide comprising the sequence Val-Ile-Val-Ile-Thr (V-I-V-I-T, SEQ ID NO:5).

The peptides that comprise the consensus amino acid sequence Val-Ile-Val-Ile-Thr, derived from the Cn-bonding motif found in NFAT proteins are peptides suitable for selectively inhibiting the transduction signal pathway of the proteins belonging to the NFAT family. Examples of these peptides are peptides having an amino acid sequence selected from SEQ ID NO: 1, 2, 3, 4, 5.

Further peptides that selectively inhibit the transduction signal pathway of the proteins belonging to the NFAT family can be selected by a person skilled in the art by using enzyme assays as described in in J. Aramburu et al. Science 1999, 285, 2129-1133.

The active molecule can be bound directly to the amphiphilic coating, for example by means of a carboxylic group present on the amphiphilic polymer, or indirectly, for example by means of a linker, with any type of chemical bond known in the art.

The linker may have a reversible bond with said active molecule, such as a disulphide bond, or an irreversible bond.

Examples of suitable linkers are 2,2-(ethylenedioxy)bis(ethylamine) (EDBE), PEG, heterobifunctional PEG, diamino PEG, ethylenediamine, and other homobifunctional or heterobifunctional spacers used commonly for solid-phase synthesis.

Figure 14 shows a general reaction scheme of conjugation of a peptide to the nanoconstruct by means of the EDBE-PDP linker.

### Biocompatible compound

In one embodiment of the invention, besides the above-described molecules, a biocompatible compound designed to modulate the bioavailability and/or biocompatibility of the constructs of the invention may also be present on the surface of the amphiphilic coating of the nanoconstructs. Such a compound is bound to the nanoparticle through said amphiphilic coating.

The compound will be preferably conjugated to the amphiphilic coating with suitable linkers, as described in the paragraph above. An example of a biocompatible compound is PEG, while an example of conjugation is by means of the EDBE linker in accordance with the same reaction schema and reagents illustrated in figure 14.

### Step b) of the method

The nanoconstructs as described above can be prepared in solutions or suspensions at the desired concentrations to be used in step b) of the method.

Such a step comprises contacting the above-described nanoconstructs, for example in solution or suspension, with an animal (for example mammal, preferably human) eukaryotic cell. Said eukaryotic cell can be selected from a cell of the immune system, for example a DC, a neutrophil or a macrophage, a lymphocyte, a cell of the nervous system, for example a neuron, tumor cells, and endothelial cells.

### Nanoconstruct for use in the treatment of a disease associated to a functional alteration of a protein localized in the cytosol

The present invention also relates to the above-described nanoconstructs in which said molecule is a molecule having pharmacological activity for use in the treatment of a disease associated to a molecular event or process localized in the cell cytosol.

Said molecule can be any molecule that has pharmacological activity linked to its interaction with a therapeutic target localized in the cell cytosol. An exemplary, but not limiting, list includes molecules having antiviral activity, such as enfuvirtides, molecules having anti-inflammatory activity, such as a peptide able to selectively inhibit the transduction signal pathway of the proteins belonging to the NFAT family, curcumin, antibodies, antitumor drugs, oligonucleotides for gene therapy, and other molecules having anti-inflammatory activity.

In a specific embodiment of the invention, said molecule with pharmacological activity is a peptide consisting of or comprising the amino acid sequence Val Ile Val Ile Thr (VIVIT).

In particular, said nanoconstruct can be used in the treatment of a disease associated to a dysfunction and/or dysregulation of a protein belonging to the NFAT family, such as inflammatory diseases. These diseases include, but are not limited to: rheumatoid arthritis, anti-inflammatory bowel disease, allogenic or xenogeneic transplant rejection, sepsis, aplastic anemia, psoriasis, edema, lupus erythematosus, type I diabetes, asthma, pulmonary fibrosis, scleroderma, dermatomyositis, Sjogren's syndrome, Kawasaki disease, Hashimoto's thyroiditis, autoimmune hemolytic anemia, idiopathic thrombocytopenia, chronic glomerulonephritis, multiple sclerosis, cystic fibrosis, chronic relapsing hepatitis, primary biliary cirrhosis, allergic rhinitis, allergic conjunctivitis, atopic dermatitis, Crohn's disease, colitis ulcerosa, colitis/inflammatory bowel syndrome, autoimmune thyroiditis, acquired immunodeficiency diseases, Alzheimer's disease, Parkinson's disease, tumors.

### Pharmaceutical compositions

The present invention also relates to a pharmaceutical composition comprising the above-described nanoconstructs and/or one or more pharmaceutically acceptable excipients, for use in the treatment of an inflammatory state.

The term "pharmaceutically acceptable excipient" denotes an excipient, adjuvant, vehicle, solvent, diluent, active agent, isotonic agent, thickening agent or emulsifier, preservative, solid binder, lubricant and the like suitable for use in a pharmaceutical composition. Remington's Pharmaceutical Sciences, sixteenth, E. W. Martin (Mack Publishing Co., Easton, Pa., 1980) describes various excipients that can be used in the formulation of pharmaceutically acceptable compositions and the techniques used for preparation thereof.

Some examples of materials that can be used as pharmaceutically acceptable excipients include, but are not limited to, ionic exchangers, aluminum, aluminum stearate, lecithin, serum proteins, such as human albumin serum, buffer substances (phosphate, sorbic acid or potassium sorbate), partial mixtures of glycerides of plant saturated fatty acids, water, salt or electrolytes, such as protamine sulphate, disodium hydrogen phosphate, sodium chloride, zinc salts, colloidal silica, magnesium trisilicate, polyvinyl pyrrolidone, polyacrylates, waxes, fatty tissue, sugars, such as lactose, glucose and sucrose; amides, such as corn starch and potato starch; cellulose and derivatives thereof, such as carboxymethylcellulose sodium, ethylcellulose and cellulose acetate; tragacanth powder; malt; gelatin; talc; excipients, such as cocoa butter and waxes for suppositories, oils, such as arachide oil, cottonseed oil, girasol oil, sesame oil, olive oil, corn oil and soy oil; glycols, such as propylene glycols or polyethylene glycols; esters, such as oleate ethyl and ethyl laurate, buffering agents, such as magnesium hydroxide and aluminum hydroxide, alginic acid, pyrogen-free water, isotonic saline solution, Ringer's solution; ethyl alcohol and saline buffer solutions with phosphate and other non-toxic compatible lubricants, such as lauryl sulphate sodium and magnesium stearate, such as coloring agents, relaxing agents, coating agents, sweeteners, flavorings and fragrances, preservatives and antioxidants may also be present in the composition according to the judgment of the person preparing the formulation.

The pharmaceutical compositions described here may also comprise at least one active ingredient different from the nanoparticles and known to be useful in the treatment of inflammatory diseases, such as corticosteroid drugs such as prednisone and dexamethasone, cytotoxic drugs such as azathioprine and cyclophosphamide, and derivatives from bacteria or fungi such as rapamycin, FK506, and cyclosporine.

The concentrations of the further one or more active ingredients in the composition can be easily established by the experts in the art on the basis of the concentrations normally used.

The compositions can be administered orally, parenterally, endovenously, by aerosol, rectally, transdermally, subcutaneously, intracisternally, intramuscularly, vaginally, intraperitoneally, topically, sublingually and intranasally and by all administration methods known to the person skilled in the art and suitable for the purposes of the present invention.

The compositions for oral administration can take the form of liquid solutions or suspensions or powders. The compositions can be presented in the form of a single dose so as to facilitate dosing.

Solid dose forms for oral administration include capsules, tablets, pills, powders and granules. In such solid forms, the active ingredient(s) is/are mixed with at least one pharmaceutically acceptable inert excipient or vehicle, for example sodium citrate or calcium phosphate and/or fillers or extenders (such as amides, lactose, sucrose, glucose, mannitol, and silicic acid); binders (such as carboxymethylcellulose, alginates, gelatin, polyvinyl pyrrolidone, sucrose); humectants (for example glycerol); disintegrating agents (such as agar-agar, calcium carbonate, potato starch or tapioca, alginic acid, certain silicates and sodium carbonate); retardants (for example paraffin); absorbance accelerators (such as compounds of quaternary ammonium); humidifying agents (such as cetylic acid and glycerol monostearate); absorbents (such as kaolin and bentonite argile); lubricants (such as talc, calcium stearate, magnesium stearate, solid polyethylene glycols, sodium lauryl sulphate) and mixtures thereof.

In the case of capsules, tablets and pills, the dosage form may also comprise buffering agents.

The solid compositions indicated above can also be used to fill capsules of hard or soft gelatin using excipients such as lactose or milk sugar and also polyethylene glycols of high molecular weight and the like. The solid dosage forms of tablets, dragees, capsules, pills and granules can be prepared with coatings such as enteric coatings and with other coating agents known in the field of pharmaceutical formulations. They can optionally be formulated so as to release the active ingredient(s) only or preferably in certain parts of the intestinal tract, optionally in a delayed manner.

Liquid forms suitable for oral administration include, but are not limited to, pharmaceutically acceptable emulsions, gels, microemulsions, solutions, suspensions, syrups and elixirs, and may contain diluents commonly used in the art. The oral liquid forms may therefore include a suitable aqueous or non-aqueous vehicle with buffers, suspending agents and dispersants, emulsifiers, solvents, colorants, aromas and the like.

For example, they may include water or other solvents, solubilizing and emulsifying agents, such as ethyl alcohol, isopropyl alcohol, ethyl carbonate, ethyl acetate, benzyl alcohol, benzyl benzoate, propylene glycols, 1,3-butylene glycols, dimethylformamides, oils (in particular cottonseed oil, groundnut oil, corn oil, germ oil, olive oil, castor oil and sesame oil), glycerol, tetrahydrofurfuryl alcohol, polyethylene glycols and fatty acid esters of sorbitan and mixtures thereof. Besides inert diluents, the oral compositions may also include adjuvants such as humectant agents, emulsifying and suspending agents, sweetening agents, flavoring agents and fragrancing agents.

The liquid forms may also be injectable solutions, for example sterile injectable aqueous or oleaginous suspensions, and can be formulated in accordance with the known technique using suitable dispersing, humectant and suspending agents. The sterile injectable preparations can also be a sterile injectable solution, suspension or emulsion in an atoxic parenterally acceptable diluent or solvent, for example a solution in 1,3-butanediol. The acceptable vehicles and solvents that can be used include water, Ringer's solution, U.S.P. and isotonic sodium chloride solution. In addition, sterile fixed oils are conventionally used as solvent or suspension media. In addition, fatty acids such as oleic acid are used in the injectable solutions.

Preservatives and other additives, such as antimicrobial additives, antioxidants, chelating agents and inert gases can also be present (Mack (1982) Remington's Pharmaceutical Sciences, 16th edition).

The injectable formulations can be sterilized, for example by means of filtration through a filter that traps the bacteria, or by incorporating sterilizing agents in the form of solid sterile compositions that can be dissolved or dispersed in sterile water or other sterile injectable media before use.

The compositions of the present invention can also be formulated for topical administration in the form of ointments, pastes, lotions, gels, powders, solutions, sprays, inhalants, eye or eardrops, or patches. The active component (or components) is mixed in sterile conditions with a pharmaceutically acceptable vehicle and any preserving agent or buffer necessary depending on requirements. Transdermal patches can be used to provide a controlled release of the compositions described here. Absorbance enhancers can also be used to increase the flow of the compound through the skin. The rate of release can be controlled by providing a membrane that controls speed or by dispersing the compound in a polymer matrix or in a gel.

The dosage can vary according to the age and general conditions of the patient, the nature and severity of the pathology or disease, and the method and type of administration. The dose must therefore take into account the particular condition to be treated, the severity of the condition to be treated, the age, weight and general physical condition of the particular patient, and also other medicines being taken by the patient, as is well known to experts in the art. In addition, it is clear that said effective quantity can be lowered or increased as required in accordance with the responses of the treated patient and/or in accordance with the assessment of the doctor prescribing the compounds of the present invention.

The term "unit dosage form" refers to a discrete physical unit containing single doses for human subjects, each single dose containing a predetermined quantity of active material calculated so as to produce the desired therapeutic effect, in combination with a suitable pharmaceutical excipient. Typical forms of single dose include phials, syringes, mono-dose bottles of the liquid composition, or pills, tablets, capsules or the like in the case of solid compositions.

The nanoconstruct can be administered in pharmaceutical form with conventional or modified (prolonged, delayed or repeated) release, parenteral large-volume release, parenteral small-volume release, single-dose release or multiple-dose release.

The pharmaceutical product containing the nanoconstruct can also be destined for veterinary use through oral, intramammary, and cutaneous administration.

Lastly, the nanoconstruct in its various formulations can also be used for research purposes.

Typically, compositions in solid form can contain a quantity of nanoconstructs between 50 and 1000 mg per unit dose, preferably from 200 to 500 mg, compositions in liquid form can contain a quantity of nanoconstructs between 10 and 200 mg per ml of composition, preferably from 40 to 100 mg per ml of composition.

### Preparation methods

The present invention also relates to a method for preparing the nanoconstructs described here, comprising the following steps:
a) prearranging a solution/suspension of inorganic nanoparticles with an outer amphiphilic polymer-based coating activated by linkers;
b) adding to said solution/suspension one or more active molecules, leaving these to react with said linkers.

The various steps of the method are described in detail below:
In step a) a solution/suspension of inorganic nanoparticles or composites (inorganic nucleus with a first organic coating) is prearranged, as described above.

The subsequent amphiphilic coating will preferably be obtained from the condensation of poly(isobutylene-alt-maleic anhydride) and dodecylamine.

The construct thus obtained is activated with a conjugation linker, such as 2,2-(ethylenedioxy)bis(ethylamine) (EDBE).

In step b), one or more active molecules are added to said activated solution/suspension, causing it to react in accordance with the reaction scheme illustrated in detail in figure 14.

The active molecule that is conjugated will preferably be a peptide capable of selectively inhibiting the transduction signal pathway of the proteins belonging to the NFAT family.

One or more further steps of washing and/or concentration can be provided between each step of the method. The steps of washing and concentration will be carried out as known to a person skilled in the art.

The present invention has been described here with reference to some of its embodiments. It is understood that other embodiments may also exist which are based on the same inventive concept, without departing from the scope of protection of the claims as specified hereinafter.

### EXAMPLES

### 1.1 Protocol for preparing nanoconstructs conjugated with the VIVIT peptide

Commercially available inorganic nanoparticles (for example AC Diagnostics, Inc.) coated with a long-chain hydrophobic surfactant (for example magnetite coated by oleic acid or oleylamine) having uniform dimensions that can vary between 3 and 50 nm were coated with an amphiphilic polymer, obtained by condensation of poly(isobutylene-maleic anhydride) and dodecylamine as described in C.-A. J. Lin et al. Small 2008, 3, 334-341. The PMA was dissolved in chloroform at a concentration of 0.5 M and added (110 µl) to the dispersion of nanoparticles, also in chloroform (at an initial concentration of 10 mg in 1.0 ml), the mixture was then homogenized, and the solvent was then evaporated at reduced pressure. The resultant solid was resuspended in sodium borate buffer of pH 12 so as to obtain a stable and homogeneous dispersion of nanoparticles (MYTS), with use of ultrasound (sonicator) where necessary. The MYTS were concentrated and washed twice with distilled water, as is well known to those skilled in the art. The solution containing MYTS was reacted with 2,2-(ethylenedioxy)-bis-(ethylamine) (EDBE) 0.05 M (3 µl) in the presence of 1-ethyl-3-(3-dimethylaminopropyl) carbodiimide hydrochloride (EDC) 0.1 M (8 µl) for at least 1.5 hours. After being washed a number of times, the nanoparticles were left under stirring for 4 hours in the presence of N-succinimidyl-3-[2-pyridyldthio]-propionate (SPDP) (300 µl at a concentration of 10 mg/ml), then the mixture was concentrated and washed twice to obtain MFN1. Lastly, the peptide with sequence NH₂-CGGGKMAGPVIVITGPHEE-COOH (SEQ ID NO:1) (13 µl at a concentration of 1 mg/ml) and PEG-SH (with molecular weight of approximately 500 Da, 10 µl at a concentration of 10 mg/ml) were added to the mixture. The mixture was stirred for 2 hours at ambient temperature, and the suspension of nanoparticles (MYTS) was then concentrated. The procedure of conjugation of the peptide is illustrated in figure 1.

The supernatant was analyzed my means of UV, and the absorbance was read at 343 nm to determine the concentration of released pyridine-2-thione with the aim of determining the quantity of peptide/PEG arranged on the MYTS nanoparticles. The nanoparticles functionalized with the peptide and PEG were then washed twice with water, and the final concentration was determined by means of UV measurement compared with a calibration line.

### 1.2 Protocol for preparing nanoconstructs conjugated with the antiviral agent enfuvirtide

Commercially available inorganic nanoparticles (for example AC Diagnostics, Inc.) coated with a long-chain hydrophobic surfactant (for example magnetite coated by oleic acid or oleylamine) having uniform dimensions that can vary between 3 and 50 nm were coated with an amphiphilic polymer, obtained by condensation of poly(isobutylene-maleic anhydride) and dodecylamine as described in C.-A. J. Lin et al. Small 2008, 3, 334-341. The PMA was dissolved in chloroform at a concentration of 0.5 M and added (110 µl) to the dispersion of nanoparticles, also in chloroform (at an initial concentration of 10 mg in 1.0 ml), the mixture was then homogenized, and the solvent was then evaporated at reduced pressure. The resultant solid was resuspended in sodium borate buffer of pH 12 so as to obtain a stable and homogeneous dispersion of nanoparticles (MYTS), with use of ultrasound (sonicator) where necessary. The MYTS were concentrated and washed twice with distilled water, as is well known to those skilled in the art. The solution containing MYTS was reacted with 2,2-(ethylenedioxy)-bis-(ethylamine) (EDBE) 0.05 M (3 µl) in the presence of 1-ethyl-3-(3-dimethylaminopropyl) carbodiimide hydrochloride (EDC) 0.1 M (8 µl) for at least 1.5 hours. After being washed a number of times, the nanoparticles were treated with enfuvirtide (SEQ ID NO:6) (5 µg dissolved in water at a concentration of 1 mg/ml) for 2 hours, then the mixture was concentrated and washed twice with water.

### 1.3 Protocol for preparing nanoconstructs conjugated with an organic fluorophore

A solution of fluoresceinamine 1.0 M (0.5 ml in DMSO) was added to a solution of PMA 0.5 M in CHCl₃ (5 ml). The mixture was reacted under stirring for one night to obtain a solution of MYTS. This solution (63 µl) was added to the solution of MYTS (4.6 mg in CHCl₃), the mixture was then homogenized, and the solvent was evaporated at reduced pressure. Sodium borate buffer was added (SBB, pH 12, 10 ml) and the suspension of nanoparticles that was concentrated in Amicon tubes was concentrated (cutoff 100 kDa) by means of centrifugation at 3500 rpm (1 hour). The fluorescent nanoparticles were washed twice, diluting with SBB, and concentrated (each cycle of centrifugation lasted for approximately 20 minutes at 3500 rpm) until a final volume of 200 µl.

### 1.4 Protocol for preparing A/O creme containing nanoconstructs conjugated, or not, with an organic fluorophore

120 µL of a suspension of MYTS or MYTS-FITC nanoparticles (40 µg/ml, H₂O) were incorporated in approximately 60 mg of a commercial base cream of continuous lipophilic phase (for example Essex, Schering-Plough S.p.A.) and were then assessed, by means of progressive solution, up to 600 mg of cream in total.

### 2. Experimentation in biological models

### 2.1 Experiments for verifying the ability of the nanoconstructs to diffuse into the cytosolic space of DCs

For the purpose of assessing whether the nanoconstructs of the present invention are able to reach the cell cytosol, a test was carried out with DCs. In particular, DCs derived from mouse bone marrow were incubated for five hours *in vitro* with the nanoconstructs prepared as described in example 1, and their ability to diffuse into the cytosolic space was measured by means of TEM (stands for transmission electron microscopy). It was observed that, partly penetrating the cell membrane directly and partly escaping conventional compartmentalization by means of endosomes, the nanoconstructs are able to diffuse into the cytosolic space (figure 1). The target Cn protein was basically localized in the cytosol, the ability to reach the cytosol therefore being fundamental for the active transport of the peptide having the sequence VIVIT to the target protein.

### 2.2 Inhibition of the transduction signal pathway of NFAT in vitro

The nanoconstructs were prepared as described in example 1, inhibiting the signaling pathway of NFAT *in vitro.*

In order to assess whether nanoconstructs are able to inhibit the transduction signal pathway of NFAT, the following experiments were carried out *in vitro.* After exposure to LPS, the DCs derived from bone marrow were able to produce various inflammatory cytokines, including IL-2 and TNFα. IL-2 was strictly NFAT-dependent, whereas the production of TNFα was NF-kB-dependent and NFAT-independent. IL-2 and TNFα are therefore valid markers respectively of the activation pathway of NFAT and NF-kB. After two hours of pretreatment with the nanoconstructs obtained as described in example 1, the DCs derived from bone marrow were stimulated with LPS and the quantity of released cytokines was measured using the ELISA method 18 hours later. As shown in figure 2, the nanoconstructs completely block IL-2, but not the production of TNFα, indicating that these nanoparticles are able to selectively inhibit, effectively, *in vitro* the transduction signal pathway of NFAT in cells of the innate immune system.

### 2.3 Inhibition of the transduction pathway of NFAT in vivo

Mutant mice (obtained from laboratories of Ronald H. Schwartz, NIH Bethesda) were used for this experiment. In this mouse model, the GFP protein was inserted into the second exon of the gene IL-2, and therefore had the same regulation of IL-2. As mentioned before, IL-2 is a marker of NFAT activation. If the mice genetically modified with regard to IL-2/GFP are treated with LPS, a significant percentage of GFP+ neutrophils (Ly6G+ cells) can be measured in the spleen two hours after treatment. To verify whether the nanoconstructs of the present invention are able to inhibit the signal pathway of NFAT *in vivo,* mutant mice were injected endovenously with the nanoconstructs as prepared in example 1 two hours before the injection of LPS. As shown in figure 3, the pretreatment with the nanoconstructs has a strong impact on the ability of the neutrophils to produce GFP in an NFAT-dependent manner, indicating that the nanoconstructs are able to inhibit the transduction signal pathway of NFAT *in vivo.*

### 2.4. Inhibition of the formation of edema in NFAT-dependent manner in a model of inflammation induced by LPS in vivo.

The formation of edema is one of the first steps in the generation of the inflammatory process and is a fundamental process for the local accumulation of inflammatory mediators. Local swelling is also relevant for the transport of the free antigen at the draining lymph nodes. The antigens present in the inflammatory tissues are transported to the lymph nodes in two successive phases. In the first phase of arrival, the antigens in free form enter the afferent lymphatic vessels and reach the draining lymph node. In the second phase, which occurs later, the antigens are transported attached to the DCs. The increase in interstitial pressure caused by the formation of edema forces part of the interstitial fluid into the afferent lymphatic vessels and promotes the entry of the free antigen into the afferent lymphatic vessels themselves and the arrival of the free antigen at the draining lymph nodes.

In previous studies, we have demonstrated that the gene Ptges1, expressed by DCs following exposure to LPS, is regulated by NFAT. Ptges1 is a protein called microsomal prostaglandin 1 E synthase 1 (mPGES-1). mPGES-1 together with cyclooxygenase-2 (COX-2) and phospholipase A2 (cPLA2), coordinates a biosynthesis process that leads to the production of PGE2, one of the more versatile prostanoids involved in the regulation of many physiological and physiopathological responses, including the formation of local edema in the inflammatory process through vasodilation. The inventors of the present invention have observed that the formation of edema induced by LPS is DC- and NFAT-dependent. DCs, thanks to the regulation of the formation of edema through NFAT following exposure to LPS and thanks to the production of PGE2, therefore regulate the arrival of the free antigen at the draining lymph nodes. The NFAT/mPGES-1 pathway therefore represents a possible target for anti-inflammatory therapies.

To assess whether the nanoparticles of the present invention are able to inhibit *in vivo* the formation of LPS-induced edema, the mice were pretreated, or not, with the nanoconstructs of example 1 or FK506 as control sub-cutaneously for 2 hours prior to the injection of LPS in the paw. As shown in figure 4, the formation of NFAT-dependent edema induced by LPS was significantly inhibited by the nanoconstructs of the present invention, confirming that these nanoparticles can effectively inhibit *in vivo* the activation of NFAT mediated by Cn.

### 2.5 Inhibition of the development of inflammatory arthritis in the mouse model of arthritis induced by anti-collagen antibodies of the IL/LPS type

Rheumatoid arthritis (AR) is a chronic, debilitating systemic autoimmune disease characterized by an immunological inflammatory reaction of the mesenchymal tissue and of the synovial fluid accompanied by polyarticular synovitis. Over time, it leads to the progressive destruction of the articular and periarticular structures. NFAT is substantially active in hemapoietic and mesenchymal cells in inflamed joints and it has recently been demonstrated that the NFATc2 and c3 isoforms have a role in the regulation of the symmetry and intensity of the synovitis in mouse models of arthritis in which the inflammatory process is lymphocyte(adaptive immunity)-independent. This suggests a completely new role of NFAT molecules in innate immunity, relevant for the pathogenesis of rheumatoid arthritis. To verify whether the nanoconstructs of the present invention are able to inhibit the development of inflammatory arthritis, a model of arthritis induced by anti-collagen antibody of the IL/LPS type was used. In particular, six C57BL6 mice were treated on days -1, 0, +1, +3, +6, +9, + 12 with the nanoconstructs of example 1. As shown in figure 5, the nanoconstructs inhibit the development of arthritis very effectively.

### 2.6 Inhibition of skin transplant rejection

To verify whether the nanoconstructs of the present invention are able to inhibit transplant rejection, a skin transplant model was used. Normally, male skin transplants into female mice are rejected within approximately three weeks in 90-100% of mice. The female mice were therefore subject to transplant of strips of male skin and were treated every three days with the nanoconstructs of example 1 from the day before transplant. The state of the transplant was inspected every day. After four weeks, the skin transplants were accepted in 90% of female mice with the nanoconstructs bearing the VIVIT peptide, whereas the transplants were rejected in 80% of mice treated with the control nanoconstructs (figure 6). This experiment indicates that the VIVIT nanoconstructs can effectively inhibit immune responses against minor histocompatibility antigens *in vivo.*

### 2.7 Comparative experiments of inhibition of the transduction signal pathway of NFAT in vitro

DCs produce IL-2 in an NFAT-dependent manner if stimulated with LPS. To assess the efficacy of the VIVIT peptide, DCs derived from bone marrow were transduced with a sequence coding the VIVIT peptide fused with the GFP protein through use of a retroviral vector.

As shown in figure 8, the peptide VIVIT expressed within the cell was able to completely block the expression of IL-2, whereas it was unable to block the expression of TNF-α, a cytokine produced by the dendritic cells in response to LPS in an NFAT-independent manner.

To obtain the pharmacological blocking of the function of NFAT in innate immunity cells through a procedure that can be used *in vivo* and that does not require the infection or transfection of the cells, we proceeded by analyzing various possible delivery systems in order to identify the system able to transfer the VIVIT peptide in the cell cytosol where the activity of NFAT must be blocked.

Since DCs are phagocyte cells, we firstly used the non-modified peptide, assuming that this could reach the cell cytosol once entered into the endosomal compartment. We then carried out co-cultures of DCs with the VIVIT peptide, and the inhibition of NFAT was analyzed by measuring the production of IL-2 following stimulation with LPS. As shown in figure 9, the use of the free, non-modified VIVIT peptide did not make it possible to interfere with the activity of NFAT. A modified VIVIT peptide was then assessed, to which a hydrophobic 11-arginine tail had been attached. It has, in fact, been described in the literature that this modification can allow entry into the cells. However, this modification also did not produce the expected result and it was not possible to observe any inhibition of the NFAT pathway induced in DCs by LPS (figure 9).

Phospholipid-based liposomes obtained from preparations of liposomes with VIVIT sequences included were used. Such preparations were incubated with DCs before stimulation with LPS. Even this system, however, did not prove to be effective in inhibiting the function of NFAT within cells (figure 10). Moreover, there was the further disadvantage concerning the impossibility of determining the loading of the active ingredient (the VIVIT peptide), which would have lead to inconsistencies in the results of each preparation.

Since the liposomal formulations did not demonstrate any level of efficacy in the transfer of the VIVIT peptide within DCs, we analyzed magnetic nanoparticles as a new delivery system. The VIVIT peptide was then conjugated with the linker described in figure 11A to nanoparticles formed of a magnetic core of crystalline magnetite measuring approximately 8 nm in diameter. As described before for the liposomal formulations, the nanoparticles conjugated directly with the VIVIT peptide were incubated with the DCs before they were stimulated with LPS. In this case too, it was not possible to observe any inhibition of the NFAT pathway induced by LPS in the DCs (figure 11B).

### 2.8 Experiments for verifying the ability of the nanoconstructs conjugated to PEG to diffuse into the cytosolic space of dendritic cells

5 million DCs derived from bone marrow were plated in full medium in 100 mm plates and were incubated for 5 hours with MYTS nanoparticles (25 µg/ml). The DCs were then separated, washed with PBS, and fixed with glutaraldehyde 2.5%. The cells were then post-fixed in osmium tetroxide 1.5%, and were then dehydrated in ascending scale of alcohol and immersed in epon.

The samples were cut by microtome and stained with uranyl acetate and lead citrate. The sections were then measured by TEM (figure 13).

### 2.9 Experiments for verifying the ability of the nanoconstructs conjugated to the antiviral agent enfuvirtide to diffuse into the cytosolic space of neuronal cells

Nude balb/c mice were injected endovenously with a solution containing just the antiretroviral drug enfuvirtide (known for being poorly permeable through the BBB) labeled with the fluorochrome AlexaFluor660 (AF660), or enfuvirtide (still labeled with AF660) conjugated to the nanoparticles (MYTS). After 1 hour, during which time the mice were kept asleep on their backs with a magnet applied to the skull, the brain samples isolated from the mice treated with MYTS/enfuvirtide or free enfuvirtide were cryosectioned. Then, a staining of the neurons was carried out with the fluorescent probe NeuroTrace (which stains the substance Nissi, which is abundant in neuronal cells and is formed from ribosomal RNA associated with the endoplasmic reticulum). The nuclei of the nervous cells were also labeled with DAPI, and the signal of AF660 associated with the drug, conjugated or not to the nanoparticles, was observed by confocal microscopy. The results show an accumulation of the fluorescent drug in the neurons, only in the case of the drug conjugated to the MYTS nanoparticles (figure 12).

### 2.10 Experiments for verifying the ability of a cream containing nanoconstructs conjugated to fluorescent molecule to diffuse through the skin in vivo

Dispersions containing high concentrations of nanoparticles labeled, and not, with fluorophore (MYTS-FITC and MYTS, respectively), were incorporated in an approximately double quantity of base cream and were then added to the rest of the base cream in successive dilutions, mixing until complete homogenization was achieved. The aforementioned preparations were then applied for three consecutive days to the skin of C57BL/6 mice, shaved beforehand (area of 1 cm²). The mice were killed, and a unicellular suspension was produced from the region of treated skin. The cells were then analyzed by FACS to determine the uptake in cells of hematopoietic origin (CD45+), and not (CD45-). As can be seen from the figure, the nanoparticles are absorbed by both groups of cells.

### DESCRIPTION OF THE SEQUENCES

**Table of sequences**

| | |
|---|---|
| SEQ ID NO:1 | CGGGKMAGPVIVITGPHEE |
| SEQ ID NO:2 | *CGGVIVIT* |
| SEQ ID NO:3 | CGGGKMAGPHPVIVITGPHEE |
| SEQ ID NO:4 | CGGGMAGPVIVITGPHEE |
| SEQ ID NO:5 | VIVIT |
| SEQ ID NO:6 | YTSLIHSLIEESQNQQEKNE QELLELDKWASLWNWF |
| SEQ ID NO:7 (control sequence) | CGGGKMAGPPHIVEETGPHVI |

### SEQUENCE LISTING

<110> UNIVERSITA' DEGLI STUDI DI MILANO - BICOCCA et al.
<120> NANOCONSTRUCTS WITH PHARMACOLOGICAL ACTIVITY
<160> 7
<170> BiSSAP 1.0
<210> 1
   <211> 19
   <212> PRT
   <213> Homo sapiens
<220>
   <221> SOURCE
   <222> 1..19
   <223> /mol_type="protein" /organism="Homo sapiens"
<400> 1
   Cys Gly Gly Gly Lys Met Ala Gly Pro Val Ile Val Ile Thr Gly Pro
   1               5                   10                  15
   His Glu Glu
<210> 2
   <211> 8
   <212> PRT
   <213> Homo sapiens
<220>
   <221> SOURCE
   <222> 1..8
   <223> /mol_type="protein" /organism="Homo sapiens"
<400> 2
   Cys Gly Gly Val Ile Val Ile Thr
   1               5
<210> 3
   <211> 21
   <212> PRT
   <213> Homo sapiens
<220>
   <221> SOURCE
   <222> 1..21
   <223> /mol_type="protein" /organism="Homo sapiens"
<400> 3
   Cys Gly Gly Gly Lys Met Ala Gly Pro His Pro Val Ile Val Ile Thr
   1               5                   10                  15
   Gly Pro His Glu Glu
               20
<210> 4
   <211> 18
   <212> PRT
   <213> Homo sapiens
<220>
   <221> SOURCE
   <222> 1..18
   <223> /mol_type="protein"
<400> /organism="Homo sapiens"
<210> 5
   <211> 5
   <212> PRT
   <213> Homo sapiens
<220>
   <221> SOURCE
   <222> 1..5
   <223> /mol_type="protein" /organism="Homo sapiens"
<400> 5
   Val Ile Val Ile Thr
   1               5
<210> 6
   <211> 36
   <212> PRT
   <213> Homo sapiens
<220>
   <221> SOURCE
   <222> 1..36
   <223> /mol_type="protein" /organism="Homo sapiens"
<400> 6
   Tyr Thr Ser Leu Ile His Ser Leu Ile Glu Glu Ser Gln Asn Gln Gln
   1               5                   10                  15
   Glu Lys Asn Glu Gln Glu Leu Leu Glu Leu Asp Lys Trp Ala Ser Leu
               20                  25                  30
   Trp Asn Trp Phe
           35
<210> 7
   <211> 21
   <212> PRT
   <213> Homo sapiens
<220>
   <221> SOURCE
   <222> 1..21
   <223> /mol_type="protein" /organism="Homo sapiens"
<400> 7 SEQUENCE LISTING
<110> UNIVERSITA' DEGLI STUDI DI MILANO - BICOCCA et al.
<120> NANOCONSTRUCTS WITH PHARMACOLOGICAL ACTIVITY
<160> 7
<170> BiSSAP 1.0
<210> 1
   <211> 19
   <212> PRT
   <213> Homo sapiens
<220>
   <221> SOURCE
   <222> 1..19
   <223> /mol_type="protein" /organism="Homo sapiens"
<400> 1
   Cys Gly Gly Gly Lys Met Ala Gly Pro Val Ile Val Ile Thr Gly Pro
   1               5                   10                  15
   His Glu Glu
<210> 2
   <211> 8
   <212> PRT
   <213> Homo sapiens
<220>
   <221> SOURCE
   <222> 1..8
   <223> /mol_type="protein" /organism="Homo sapiens"
<400> 2
   Cys Gly Gly Val Ile Val Ile Thr
   1               5
<210> 3
   <211> 21
   <212> PRT
   <213> Homo sapiens
<220>
   <221> SOURCE
   <222> 1..21
   <223> /mol_type="protein" /organism="Homo sapiens"
<400> 3
   Cys Gly Gly Gly Lys Met Ala Gly Pro His Pro Val Ile Val Ile Thr
   1               5                   10                  15
   Gly Pro His Glu Glu
               20
<210> 4
   <211> 18
   <212> PRT
   <213> Homo sapiens
<220>
   <221> SOURCE
   <222> 1..18
   <223> /mol_type="protein" /organism="Homo sapiens"
<400> 4
   Cys Gly Gly Gly Met Ala Gly Pro Val Ile Val Ile Thr Gly Pro His
   1               5                   10                  15
   Glu Glu
<210> 5
   <211> 5
   <212> PRT
   <213> Homo sapiens
<220>
   <221> SOURCE
   <222> 1..5
   <223> /mol_type="protein" /organism="Homo sapiens"
<400> 5
   Val Ile Val Ile Thr
   1               5
<210> 6
   <211> 36
   <212> PRT
   <213> Homo sapiens
<220>
   <221> SOURCE
   <222> 1..36
   <223> /mol_type="protein" /organism="Homo sapiens"
<400> 6
   Tyr Thr Ser Leu Ile His Ser Leu Ile Glu Glu Ser Gln Asn Gln Gln
   1               5                   10                  15
   Glu Lys Asn Glu Gln Glu Leu Leu Glu Leu Asp Lys Trp Ala Ser Leu
               20                  25                  30
   Trp Asn Trp Phe
           35
<210> 7
   <211> 21
   <212> PRT
   <213> Homo sapiens
<220>
   <221> SOURCE
   <222> 1..21
   <223> /mol_type="protein" /organism="Homo sapiens"
<400> 7
   Cys Gly Gly Gly Lys Met Ala Gly Pro Pro His Ile Val Glu Glu Thr
   1               5                   10                  15
   Gly Pro His Val Ile
               20

## Claims

1. A nanoconstruct comprising an inorganic nanoparticle with an amphiphilic polymer-based external coating and a molecule with pharmacological activity bound to said nanoparticle through the amphiphilic polymer for use in the treatment of a disease associated to a molecular event or process localized in the cell cytosol, wherein the active molecule is a peptide selected from SEQ ID NO: 6, nuclear localization sequences, or a peptide comprising the sequence SEQ ID NO: 5.

2. The nanoconstruct according to claim 1, wherein the inorganic nanoparticle is a metal or a metal oxide, for use according to claim 1.

3. The nanoconstruct according to claim 1, wherein the nanoparticle is comprised of Au, Ag, Fe, Mn, or of γ-Fe₂O₃, Fe₃O₄, MnFe₂O₄, CoFe₂O₄, MnO, TiO₂, SiO₂, CeO₂, or of a quantum dots selected from CdSe, CdS, CdSe/ZnS, for use according to claim 1.

4. The nanoconstruct according to any one of the claims 1 to 3 , wherein said amphiphilic polymer is comprised of block copolymers, for use according to claim 1

5. The nanoconstruct according to claim 14, wherein the amphiphilic polymer is obtained by condensation of poly(isobutylene-*alt*-maleic anhydride) and dodecylamine for use according to claim 1.

6. The nanoconstruct according to any one of the claims 1 to 5, wherein said active molecule is bound to the amphiphilic coating also by a linker with a reversible or irreversible bond, for use according to claim 1.

7. The nanoconstruct according to any one of the claims 1 to 6, wherein said linker is reversible and comprises a disulphide bridge, for use according to claim 1.

8. The nanoconstruct according to any one of the claims 1 to 7 wherein said active molecule is selected from an oligopeptide or polypeptide, a saccharide, oligosaccharide or polysaccharide, a DNA, RNA, siRNA, miRNA sequence or a synthetic polymer or a pharmacologically active molecule, for use according to claim 1.

9. The nanoconstruct according to claim 1, wherein the active substance is a peptide having SEQ ID NO: 1, 2, 3, 4, 5, for use according to claim 1.

10. The nanoconstruct according to any one of the claims 1 to 9, comprising a second biocompatible compound, bound to the nanoparticle through the amphiphilic polymer, for use according to claim 1.

11. The nanoconstruct according to any one of the claims 1 to 10, wherein the sizes of said nanoparticles are comprised between 5 and 100 nanometers, for use according to claim 1.

12. The nanoconstruct according to any one of the claims 1 to 11 for use in the treatment of a disease associated to a functional alteration of a protein localized in the cell cytosol or nucleus.

13. The nanoconstruct according to any one of the previous claims for use in a treatment according to the previous claims, said treatment consisting in selectively inhibiting the signal transduction pathway of the proteins belonging to the NFAT family.

14. The nanoconstruct according to claim 13 for use according to claim 13, wherein said disease is an inflammatory state.

15. The nanoconstruct according to claim 14, for use according to claim 14, wherein said disease is selected from: rheumatoid arthritis, inflammatory bowel disease, allogeneic or xenogeneic transplantation rejection, sepsis, aplastic anemia, psoriasis, edema, lupus erythematosus, type I diabetes, asthma, pulmonary fibrosis, scleroderma, dermatomyositis, Sjogren's syndrome, Kawasaki disease, Hashimoto's thyroiditis, autoimmune hemolytic anemia, idiopathic thrombopenia, chronic glomerulonefritis, multiple sclerosis, cystic fibrosis, chronic relapsing hepatitis, primary biliary cirrhosis, allergic rhinitis, allergic conjunctivitis, atopic dermatitis, Crohn's disease, ulcerative colitis, colitis/inflammatory bowel syndrome, autoimmune thyroiditis, acquired immunodeficiency diseases, Alzheimer disease, Parkinson disease, tumors.

16. A pharmaceutical composition comprising a nanoconstruct according to any one of the claims 1 to 15 and one or more excipients for use in the treatment of a disease or of an inflammatory state.

17. The composition according to claim 16 for use according to claim 16, wherein the disease is selected from: rheumatoid arthritis, inflammatory bowel disease, allogeneic or xenogeneic transplantation rejection, sepsis, aplastic anemia, psoriasis, edema, lupus erythematosus, type I diabetes, asthma, pulmonary fibrosis, scleroderma, dermatomyositis, Sjogren's syndrome, Kawasaki disease, Hashimoto's thyroiditis, autoimmune hemolytic anemia, idiopathic thrombopenia, chronic glomerulonefritis, multiple sclerosis, cystic fibrosis, chronic relapsing hepatitis, primary biliary cirrhosis, allergic rhinitis, allergic conjunctivitis, atopic dermatitis, Crohn's disease, ulcerative colitis, colitis/inflammatory bowel syndrome, autoimmune thyroiditis, acquired immunodeficiency diseases, Alzheimer disease, Parkinson disease, tumors.

18. The pharmaceutical composition according to any one of the claims 16 or 17 suitable for oral, intravenous, intramuscular, subcutaneous, transdermal, nasal, vaginal, rectal or intraperitoneal administration, for use according to claims 16 or 17.

19. A method for the preparation of a nanoconstruct according to any one of the claims 1 to 11, comprising the following steps:
a) prearranging a solution/suspension of inorganic nanoparticles with an external coating of an amphiphilic polymer activated by a linker;
b) adding to said solution/suspension one or more active molecules, letting it react with said linker.

20. The method according to claim 19, **characterized in that** the amphiphilic polymer is activated by a linker creating a reversible or irreversible bond.

21. The method according to claim 20, wherein said reversible bond is obtained according to the scheme of figure 14.

## Patentansprüche

1. Nanokonstrukt, das ein anorganisches Nanopartikel mit einer amphiphilen externen Beschichtung auf Polymerbasis und ein Molekül mit einer pharmakologischen Aktivität, das durch das amphiphile Polymer an das Nanopartikel gebunden ist, umfasst, zur Verwendung bei der Behandlung einer Krankheit, die mit einem molekularen Ereignis oder Prozess, der im Cytosol der Zelle angeordnet ist, verknüpft ist, wobei das aktive Molekül ein Peptid ist, das aus der folgenden Gruppe ausgewählt wird: SEQ ID No: 6, Kernlokalisierungssequenzen, oder ein Peptid ist, das die Sequenz SEQ ID NO: 5 umfasst.

2. Nanokonstrukt gemäß Anspruch 1, wobei das anorganische Nanopartikel ein Metall oder ein Metalloxid ist, zur Verwendung gemäß Anspruch 1.

3. Nanokonstrukt gemäß Anspruch 1, wobei das Nanopartikel umfasst: Au, Ag, Fe, Mn oder aus γ-Fe₂O₃, Fe₃O₄, MnFe₂O₄, CoFe₂O₄, MnO, TiO₂, SiO₂, CeO₂ oder Quantenpunkte, die aus der folgenden Gruppe ausgewählt werden: CdSe, CdS, CdSe/ZnS, zur Verwendung gemäß Anspruch 1.

4. Nanokonstrukt gemäß einem der Ansprüche 1 bis 3, wobei das amphiphile Polymer aus Blockcopolymeren besteht, zur Verwendung gemäß Anspruch 1.

5. Nanokonstrukt gemäß Anspruch 4, wobei das amphiphile Polymer durch Kondensation von Poly(isobutylen-*alt*-maleinsäureanhydrid) und Dodecylamin erhalten wird, zur Verwendung gemäß Anspruch 1.

6. Nanokonstrukt gemäß einem der Ansprüche 1 bis 5, wobei das aktive Molekül an die amphiphile Beschichtung außerdem durch einen Verknüpfer mit einer reversiblen oder irreversiblen Bindung gebunden ist, zur Verwendung gemäß Anspruch 1.

7. Nanokonstrukt gemäß einem der Ansprüche 1 bis 6, wobei der Verknüpfer reversibel ist und eine Disulfidbrücke umfasst, zur Verwendung gemäß Anspruch 1.

8. Nanokonstrukt gemäß einem der Ansprüche 1 bis 7, wobei das aktive Molekül aus der folgenden Gruppe ausgewählt wird: einem Oligopeptid oder Polypeptid, einem Saccharid, Oligosaccharid oder Polysaccharid, einer DNA-, RNA-, siRNA-, miRNA-Sequenz oder einem synthetischen Polymer oder einem pharmakologisch aktiven Molekül, zur Verwendung gemäß Anspruch 1.

9. Nanokonstrukt gemäß Anspruch 1, wobei die aktive Substanz ein Peptid ist, das über SEQ ID NO: 1, 2, 3, 4, 5 verfügt, zur Verwendung gemäß Anspruch 1.

10. Nanokonstrukt gemäß einem der Ansprüche 1 bis 9, das umfasst: eine zweite biokompatible Verbindung, die an das Nanopartikel durch das amphiphile Polymer gebunden ist, zur Verwendung gemäß Anspruch 1.

11. Nanokonstrukt gemäß einem der Ansprüche 1 bis 10, wobei die Größen der Nanopartikel Größen zwischen 5 und 100 Nanometern umfassen, zur Verwendung gemäß Anspruch 1.

12. Nanokonstrukt gemäß einem der Ansprüche 1 bis 11 zur Verwendung bei der Behandlung einer Krankheit, die mit einer funktionellen Veränderung eines Proteins, das im Cytosol oder Kern der Zelle lokalisiert ist, verknüpft ist.

13. Nanokonstrukt gemäß einem der vorangehenden Ansprüche zur Verwendung bei einer Behandlung gemäß den vorliegenden Ansprüchen, wobei die Behandlung umfasst:
selektives Hemmen des Signalübertragungspfades der Proteine, die zu der NFAT-Familie gehören.

14. Nanokonstrukt gemäß Anspruch 13 zur Verwendung gemäß Anspruch 13, wobei die Krankheit ein Entzündungszustand ist.

15. Nanokonstrukt gemäß Anspruch 14 zur Verwendung gemäß Anspruch 14, wobei die Krankheit aus der folgenden Gruppe ausgewählt wird:
Gelenkrheumatismus, entzündliche Darmerkrankung, allogene oder xenogene Transplantationsabstoßung, Sepsis, unplastische Anämie, Psoriasis, Ödem, Lupus erythematodes, Typ-I-Diabetes, Asthma, Lungenfibrose, Sklerodermie, Dermatomyositis, Sjögren-Syndrom, Kawasaki-Syndrom, Hashimoto-Thyreoiditis, autoimmunhämolytische Anämie, idiopathische Thrombopenie, chronische Nierenentzündung, Multiple Sklerose, Mukoviszidose, chronische Hepatitis, Primär billiäre Zirrhose, allergischer Schnupfen, allergische Bindehautentzündung, atopische Dermatitis, Morbus Crohn, Colitis ulcerosa, Colitis/entzündliche Darmerkrankung, Autoimmunthyreopathie, erworbene Immunschwäche, Alzheimer-Krankheit, Morbus Parkinson, Tumore.

16. Pharmazeutische Zusammensetzung, die umfasst: ein Nanokonstrukt gemäß einem der Ansprüche 1 bis 15 und ein oder mehrere Hilfsstoffe zur Verwendung bei der Behandlung einer Krankheit oder eines Entzündungszustands.

17. Zusammensetzung gemäß Anspruch 16 zur Verwendung gemäß Anspruch 16, wobei die Krankheit aus der folgenden Gruppe ausgewählt wird:
Gelenkrheumatismus, entzündliche Darmerkrankung, allogene oder xenogene Transplantationsabstoßung, Sepsis, unplastische Anämie, Psoriasis, Ödem, Lupus erythematodes, Typ-I-Diabetes, Asthma, Lungenfibrose, Sklerodermie, Dermatomyositis, Sjögren-Syndrom, Kawasaki-Syndrom, Hashimoto-Thyreoiditis, autoimmunhämolytische Anämie, idiopathische Thrombopenie, chronische Nierenentzündung, Multiple Sklerose, Mukoviszidose, chronische Hepatitis, Primär billiäre Zirrhose,, allergischer Schnupfen, allergische Bindehautentzündung, atopische Dermatitis, Morbus Crohn, Colitis ulcerosa, Colitis/entzündliche Darmerkrankung, Autoimmunthyreopathie, erworbene Immunschwäche, Alzheimer-Krankheit, Morbus Parkinson, Tumore.

18. Pharmazeutische Zusammensetzung gemäß einem der Ansprüche 16 oder 17, die für eine orale, intravenöse, Intramuskuläre, subkutane, transdermale, nasale, vaginale, rektale oder intraperitoneale Verabreichung geeignet ist, zur Verwendung gemäß der Ansprüche 16 oder 17.

19. Verfahren zur Herstellung eines Nanokonstrukts gemäß einem der Ansprüche 1 bis 11, das die folgenden Schritte umfasst:
a) Bestimmen einer Lösung/Suspension von anorganischen Nanopartikeln mit einer externen Beschichtung aus einem amphiphilen Polymer, das durch einen Verknüpfer aktiviert wird;
b) Hinzufügen, zu der Lösung/Suspension, eines oder mehrerer aktiver Moleküle, wobei diese mit dem Verknüpfer reagieren können.

20. Verfahren gemäß Anspruch 19, **dadurch gekennzeichnet, dass** das amphiphile Polymer durch einen Verknüpfer aktiviert wird, der eine reversible oder irreversible Bindung erzeugt.

21. Verfahren gemäß Anspruch 20, wobei die reversible Bindung gemäß dem Schema von Abbildung 14 erhalten wird.

## Revendications

1. Nanoconstruction comprenant une nanoparticule inorganique avec un revêtement externe à base de polymère amphiphile et une molécule dotée d'une activité pharmacologique liée à ladite nanoparticule par l'intermédiaire du polymère amphiphile, destinée à une utilisation dans le traitement d'une maladie associée à un événement ou un processus moléculaire localisé dans le cytosol cellulaire, dans laquelle la molécule active est un peptide choisi parmi SEQ ID NO : 6, des séquences à localisation nucléaire, ou un peptide comprenant la séquence SEQ ID NO : 5.

2. Nanoconstruction selon la revendication 1, dans laquelle la nanoparticule inorganique est un métal ou un oxyde de métal, destinée à une utilisation selon la revendication 1.

3. Nanoconstruction selon la revendication 1, dans laquelle la nanoparticule est composée de Au, Ag, Fe, Mn, ou de γ-Fe₂O₃, Fe₃O₄, MnFe₂O₄, CoFe₂O₄, MnO, TiO₂, SiO₂, CeO₂ ou d'un point quantique choisi parmi CdSe, CdS, CdSe/ZnS, destinée à une utilisation selon la revendication 1.

4. Nanoconstruction selon l'une quelconque des revendications 1 à 3, dans laquelle ledit polymère amphiphile est composé de copolymères séquencés, destinée à une utilisation selon la revendication 1.

5. Nanoconstruction selon l'une quelconque des revendications 1 à 14, dans laquelle le polymère amphiphile est obtenu par condensation de poly(isobutylène-*alt*-anhydride maléique) et de dodécylamine, destinée à une utilisation selon la revendication 1.

6. Nanoconstruction selon l'une quelconque des revendications 1 à 5, dans laquelle ladite molécule active est liée au revêtement amphiphile également par un lieur avec une liaison réversible ou irréversible, destinée à une utilisation selon la revendication 1.

7. Nanoconstruction selon l'une quelconque des revendications 1 à 6, dans laquelle ledit lieur est réversible et comprend un pont disulfure, destinée à une utilisation selon la revendication 1.

8. Nanoconstruction selon l'une quelconque des revendications 1 à 7, dans laquelle ladite molécule active est choisie parmi un oligopeptide ou un polypeptide, un saccharide, un oligosaccharide ou un polysaccharide, une séquence d'ADN, d'ARN, d'ARNsi, de miARN ou un polymère synthétique ou une molécule pharmacologiquement active, destinée à une utilisation selon la revendication 1.

9. Nanoconstruction selon la revendication 1, dans laquelle la substance active est un peptide ayant SEQ ID NO : 1, 2, 3, 4, 5, destinée à une utilisation selon la revendication 1.

10. Nanoconstruction selon l'une quelconque des revendications 1 à 9, comprenant un second composé biocompatible, lié à la nanoparticule par l'intermédiaire du polymère amphiphile, destinée à une utilisation selon la revendication 1.

11. Nanoconstruction selon l'une quelconque des revendications 1 à 10, dans laquelle les tailles desdites nanoparticules sont comprises entre 5 et 100 nanomètres, destinée à une utilisation selon la revendication 1.

12. Nanoconstruction selon l'une quelconque des revendications 1 à 11, destinée à une utilisation dans le traitement d'une maladie associée à une modification fonctionnelle d'une protéine localisée dans le cytosol ou le noyau cellulaire.

13. Nanoconstruction selon l'une quelconque des revendications précédentes, destinée à une utilisation dans un traitement selon les revendications précédentes, ledit traitement consistant en l'inhibition sélective de la voie de transduction des signaux des protéines appartenant à la famille NFAT.

14. Nanoconstruction selon la revendication 13, destinée à une utilisation selon la revendication 13, où ladite maladie est un état inflammatoire.

15. Nanoconstruction selon la revendication 14, destinée à une utilisation selon la revendication 14, où ladite maladie est choisie parmi : la polyarthrite rhumatoïde, une maladie inflammatoire de l'intestin, le rejet de transplantation allogénique ou xénogénique, la septicémie, l'anémie aplasique, le psoriasis, l'oedème, le lupus érythémateux, le diabète de type I, l'asthme, la fibrose pulmonaire, la sclérodermie, la dermatomyosite, le syndrome de Sjögren, la maladie de Kawasaki, la thyroïdite de Hashimoto, l'anémie hémolytique auto-immune, la thrombopénie idiopathique, la glomérulonéphrite chronique, la sclérose en plaques, la fibrose kystique, l'hépatite chronique récidivante, la cirrhose biliaire primitive, la rhinite allergique, la conjonctivite allergique, la dermatite atopique, la maladie de Crohn, la rectocolite hémorragique, la colite/le syndrome inflammatoire de l'intestin, la thyroïdite auto-immune, les maladies d'immunodéficience acquise, la maladie d'Alzheimer, la maladie de Parkinson, les tumeurs.

16. Composition pharmaceutique comprenant une nanoconstruction selon l'une quelconque des revendications 1 à 15 et un ou plusieurs excipients, destinée à une utilisation dans le traitement d'une maladie ou d'un état inflammatoire.

17. Composition selon la revendication 16, destinée à une utilisation selon la revendication 16, où la maladie est choisie parmi : la polyarthrite rhumatoïde, une maladie inflammatoire de l'intestin, le rejet de transplantation allogénique ou xénogénique, la septicémie, l'anémie aplasique, le psoriasis, l'oedème, le lupus érythémateux, le diabète de type I, l'asthme, la fibrose pulmonaire, la sclérodermie, la dermatomyosite, le syndrome de Sjögren, la maladie de Kawasaki, la thyroïdite de Hashimoto, l'anémie hémolytique auto-immune, la thrombopénie idiopathique, la glomérulonéphrite chronique, la sclérose en plaques, la fibrose kystique, l'hépatite chronique récidivante, la cirrhose biliaire primitive, la rhinite allergique, la conjonctivite allergique, la dermatite atopique, la maladie de Crohn, la rectocolite hémorragique, la colite/le syndrome inflammatoire de l'intestin, la thyroïdite auto-immune, les maladies d'immunodéficience acquise, la maladie d'Alzheimer, la maladie de Parkinson, les tumeurs.

18. Composition pharmaceutique selon l'une quelconque des revendications 16 ou 17 appropriée à une administration orale, intraveineuse, intramusculaire, sous-cutanée, transdermique, nasale, vaginale, rectale ou intrapéritonéale, destinée à une utilisation selon les revendications 16 ou 17.

19. Procédé pour la préparation d'une nanoconstruction selon l'une quelconque des revendications 1 à 11, comprenant les étapes suivantes :
a) le pré-arrangement d'une solution/suspension de nanoparticules inorganiques avec un revêtement externe d'un polymère amphiphile activé par un lieur ;
b) l'addition à ladite solution/suspension d'une ou de plusieurs molécules actives, en la laissant réagir avec le ledit lieur.

20. Procédé selon la revendication 19, **caractérisé en ce que** le polymère amphiphile est activé par un lieur créant une liaison réversible ou irréversible.

21. Procédé selon la revendication 20, dans lequel ladite liaison réversible est obtenue selon le schéma de la figure 14.
